# EUROPEAN PATENT APPLICATION

(11) **EP 4 842 172 A1**
(43) Date of publication of application: **18.11.2026**
(21) Application number: 25738748.0
(22) Date of filing: 07.01.2025
(51) Int. Cl.: H10K 30/50, C07C 229/46, C07D 333/20, H10K 30/40, H10K 30/86, H10K 85/50, H10K 85/60

(54) **PHOTOELECTRIC CONVERSION ELEMENT AND PHOTOELECTRIC CONVERSION DEVICE**

(30) Priority: 11.01.2024 JP 2024002687
(71) Applicant: Canon Kabushiki Kaisha, Tokyo 146-8501 (JP)
(72) Inventor: MORI, Haruki, Tokyo 146-8501 (JP); TAKAHASHI, Naohiro, Tokyo 146-8501 (JP); SATO, Mizuki, Tokyo 146-8501 (JP); OHSAWA, Tatsuya, Tokyo 146-8501 (JP); NAKAMURA, Nobuhiro, Tokyo 146-8501 (JP)
(74) Representative: TBK
(86) International application number: PCT/JP2025/000103
(87) International publication number: WO 2025/150480

(57) **Abstract**

The present invention is directed to providing a photoelectric conversion element having improved photoelectric conversion efficiency. To solve the problem above, the present invention is a photoelectric conversion element including a first electrode, a second electrode, and a photoelectric conversion layer containing crystals with a perovskite structure, the photoelectric conversion layer being disposed between the first electrode and the second electrode, wherein a hole transporting layer containing a compound having a specific structure is disposed between the photoelectric conversion layer and the first electrode.

## Description

### [Technical Field]

The present invention relates to a photoelectric conversion element and a photoelectric conversion apparatus.

### [Background Art]

To solve problems related with depletion of fossil energy and environmental problems of the earth caused by use of fossil energy, research concerning regenerable and clean alternative energy sources such as solar energy, wind power, and water power has been actively conducted. Among these, concerns on solar batteries which directly convert sunlight into electrical energy have been increasing. Here, the solar battery means a battery which generates current-voltage using the photovoltaic effect of absorbing light energy from sunlight and generating electrons and holes.

Currently, n-p diode type silicon (Si) single-crystal based solar batteries having a light energy conversion efficiency of more than 20% are widely known, and are actually used in solar energy generation. However, these need a high temperature treatment step, and the material itself is expensive, which leads to a problem of high cost per unit electric power. Moreover, these also have a problem of supplyability due to the silicon resource.

In contrast, solar batteries in which an organic material is used (hereinafter, referred to as "organic solar batteries") do not need a high temperature treatment step, and can be produced by a so-called roll to roll method using sheet-shaped substrates, so that cost can be reduced. However, for practical use of such organic solar batteries, a further improvement in power generation efficiency and durability has been desired. In order to enhance the function of selectively transporting holes to improve the photoelectric conversion efficiency, development of a hole-transporting material used in a hole transporting layer has been proceeded. For example, in PTL 1 and NPL 1, an improvement in photoelectric conversion efficiency by disposing a hole transporting layer containing a compound having a specific structure is described. In addition, perovskite solar batteries having crystals with a perovskite structure as the photoelectric conversion layer have high photoelectric conversion properties, and thus, development thereof has been particularly proceeded for practical use of the organic solar battery.

### [Citation List]

### [Patent Literature]

PTL 1: International Publication No. WO2022/153962

### [Non Patent Literature]

NPL 1: J. Kumar, et al, RSC Adv., 2013, 3, 15626

### [Summary of Invention]

### [Technical Problem]

According to the research by the present inventors, photoelectric conversion efficiency was insufficient in the photoelectric conversion elements including a photoelectric conversion layer containing crystals with a perovskite structure in which the compounds disclosed in PTL 1 and NPL 1 are used in the hole transporting layer.

Accordingly, the present invention is directed to providing a photoelectric conversion element having photoelectric conversion efficiency improved by using a compound having a specific structure in a hole transporting layer. Moreover, the present invention is directed to providing a photoelectric conversion apparatus having photoelectric conversion efficiency improved by using a compound having a specific structure in a hole transporting layer.

### [Solution to Problem]

These aspects are achieved by the present invention shown below. In other words, the present invention is a photoelectric conversion element including a first electrode, a second electrode, and a photoelectric conversion layer containing crystals with a perovskite structure, the photoelectric conversion layer being disposed between the first electrode and the second electrode, wherein a hole transporting layer containing a compound represented by the following formula (1) is disposed between the photoelectric conversion layer and the first electrode. 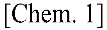 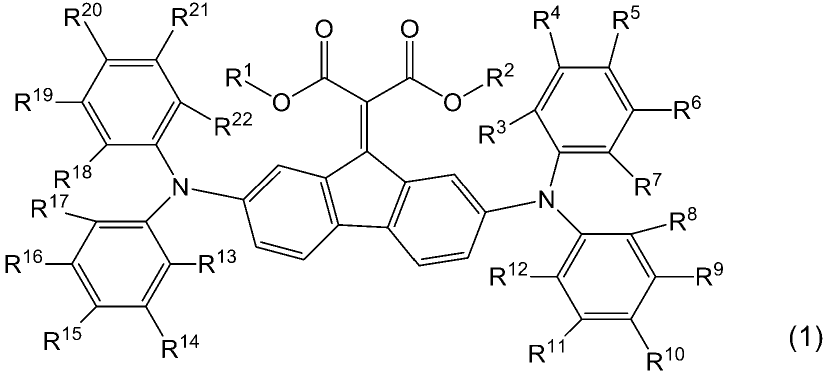 (wherein R¹ and R² each represent a linear or branched alkyl group having 1 to 6 carbon atoms, and R³ to R²² each independently represent a hydrogen atom, a trimethylsilyl group, a linear or branched alkyl group having 1 to 20 carbon atoms and optionally having a substituent, a linear or branched alkenyl group having 2 to 20 carbon atoms and optionally having a substituent, a cycloalkyl group having 3 to 10 carbon atoms and optionally having a substituent, an alkoxy group having 1 to 20 carbon atoms and optionally having a substituent, a cycloalkoxy group having 3 to 10 carbon atoms and optionally having a substituent, an alkylthio group having 1 to 18 carbon atoms and optionally having a substituent, an amino group having an alkyl group having 1 to 20 carbon atoms and optionally having a substituent, an aromatic hydrocarbon group having 6 to 36 carbon atoms and optionally having a substituent, or a heterocyclic group having 5 to 36 ring-forming atoms and optionally having a substituent; and the substituent which each of the functional groups optionally has is a halogeno group, a linear or branched alkyl group having 1 to 20 carbon atoms, an alkoxy group having 1 to 20 carbon atoms, an alkylthio group having 1 to 18 carbon atoms, an amino group having an alkyl group having 1 to 20 carbon atoms, an aromatic hydrocarbon group having 6 to 36 carbon atoms, or a heterocyclic group having 5 to 36 ring-forming atoms.)

Moreover, the present invention is a photoelectric conversion apparatus including the above-mentioned photoelectric conversion element.

### [Advantageous Effects of Invention]

According to the present invention, a photoelectric conversion element and a photoelectric conversion apparatus having improved photoelectric conversion efficiency can be provided.

### [Brief Description of Drawings]

[Fig. 1]

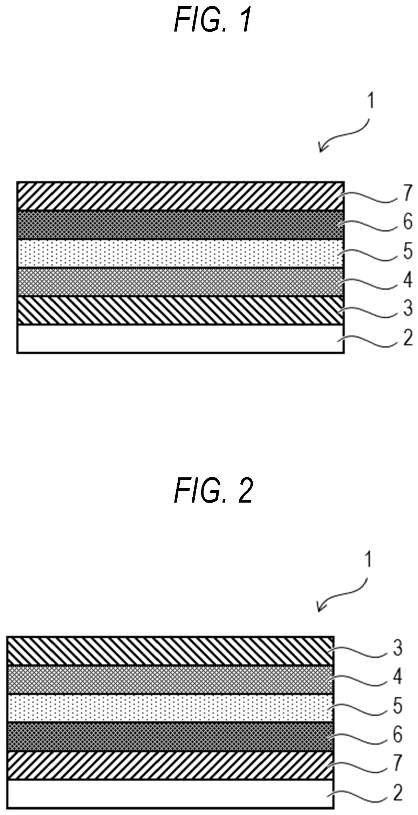

   Fig. 1 is one example of a cross-sectional schematic view of a photoelectric conversion element according to one embodiment of the present invention in the thickness direction.
[Fig. 2]
   Fig. 2 is another example of a cross-sectional schematic view of the photoelectric conversion element according to one embodiment of the present invention in the thickness direction.
[Fig. 3]

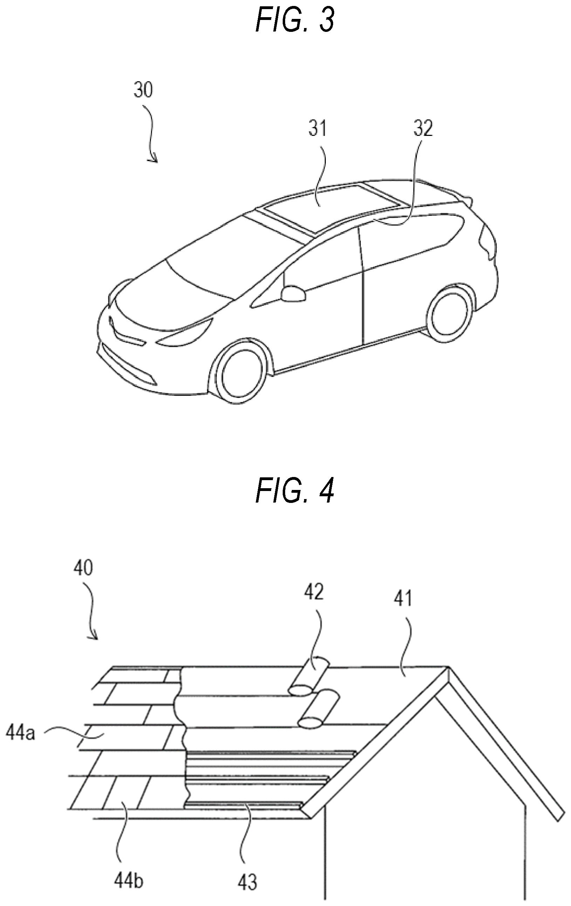

   Fig. 3 is a perspective view schematically illustrating one embodiment of a moving body including the photoelectric conversion element according to the present invention.
[Fig. 4]
   Fig. 4 is a perspective view schematically illustrating one embodiment of a construction material including the photoelectric conversion element according to the present invention.

### [Description of Embodiments]

### <Embodiment>

An embodiment relates to a photoelectric conversion element.

The photoelectric conversion element according to the present invention is a photoelectric conversion element including a first electrode, a second electrode, and a photoelectric conversion layer containing crystals with a perovskite structure, the photoelectric conversion layer being disposed between the first electrode and the second electrode, wherein a hole transporting layer containing a compound represented by the following formula (1) is disposed between the photoelectric conversion layer and the first electrode:

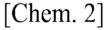

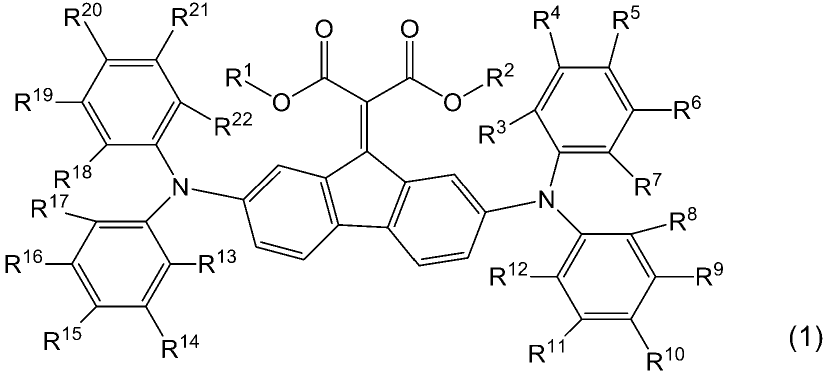

In the formula (1), R¹ and R² each represent a linear or branched alkyl group having 1 to 6 carbon atoms, and R³ to R²² each independently represent a hydrogen atom, a trimethylsilyl group, a linear or branched alkyl group having 1 to 20 carbon atoms and optionally having a substituent, a linear or branched alkenyl group having 2 to 20 carbon atoms and optionally having a substituent, a cycloalkyl group having 3 to 10 carbon atoms and optionally having a substituent, an alkoxy group having 1 to 20 carbon atoms and optionally having a substituent, a cycloalkoxy group having 3 to 10 carbon atoms and optionally having a substituent, an alkylthio group having 1 to 18 carbon atoms and optionally having a substituent, an amino group having an alkyl group having 1 to 20 carbon atoms and optionally having a substituent, an aromatic hydrocarbon group having 6 to 36 carbon atoms and optionally having a substituent, or a heterocyclic group having 5 to 36 ring-forming atoms and optionally having a substituent. The substituent which each of the functional groups optionally has is a halogeno group, a linear or branched alkyl group having 1 to 20 carbon atoms, an alkoxy group having 1 to 20 carbon atoms, an alkylthio group having 1 to 18 carbon atoms, an amino group having an alkyl group having 1 to 20 carbon atoms, an aromatic hydrocarbon group having 6 to 36 carbon atoms, or a heterocyclic group having 5 to 36 ring-forming atoms.

As a result of research, the present inventors have found that a photoelectric conversion element having improved photoelectric conversion efficiency is obtained by disposing a hole transporting layer containing a compound represented by the formula (1) between the photoelectric conversion layer and the first electrode. In the present invention, although the reason why a photoelectric conversion element having improved photoelectric conversion efficiency is obtained is not clarified in detail, the following reason can be considered.

The compound represented by the formula (1) has a structure having the characteristics (A) and (B) below.
(A) A nitrogen atom is directly bonded to the carbon atoms at the 2-position and 7-position of the fluorene skeleton.
(B) The α-carbon atom of a malonic acid ester is bonded to the carbon atom in the 9-position of the fluorene skeleton through a carbon-carbon double bond.

DFT calculations suggest that the electron distribution of the highest occupied molecular orbital (HOMO) is spread across the molecule by the structure having the characteristic (A) when such a structure is contained in the compound. It is considered that the electron distribution of the HOMO spread across the molecule leads to an enhancement in hole transportation ability in the hole transporting layer.

DFT calculations also suggest that the dipole moment of the molecule is increased by the structure having the characteristic (B) when such a structure is contained in the compound, which leads to a high polarity thereof. It is considered that by using such a hole-transporting compound having a high polarity in the hole transporting layer, interaction between the hole transporting layer and the photoelectric conversion layer is improved, therefore increasing migration efficiency of the holes generated in the photoelectric conversion layer to the hole transporting layer.

As in the mechanism described above, the effects of the present invention can be achieved by synergistic effects caused by the characteristics (A) and (B).

In the formula (1), it is preferred that R³ to R²² each independently represent a hydrogen atom, a linear or branched alkyl group having 1 to 20 carbon atoms and optionally having a substituent, an alkoxy group having 1 to 20 carbon atoms and optionally having a substituent, or an amino group having an alkyl group having 1 to 20 carbon atoms and optionally having a substituent, and it is more preferred that R⁵, R¹⁰, R¹⁵, and R²⁰ each independently represent an alkoxy group having 1 to 20 or less carbon atoms and optionally having a substituent. When such substituents are contained, the photoelectric conversion efficiency is improved.

Hereinafter, specific examples (Exemplified compounds 1-1 to 1-12) of the compound represented by the formula (1) will be shown, but the present invention is not limited to these.

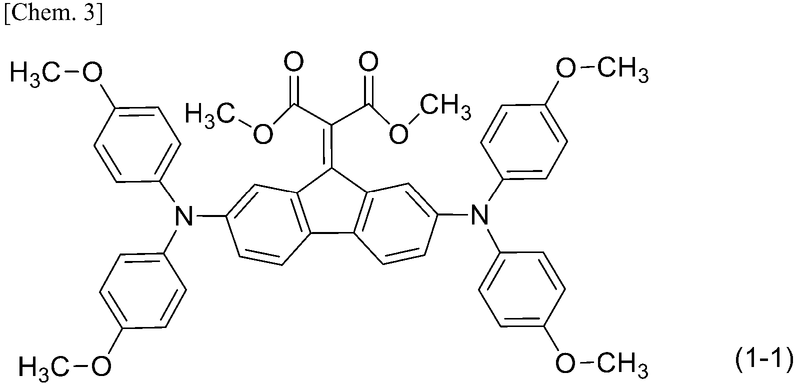

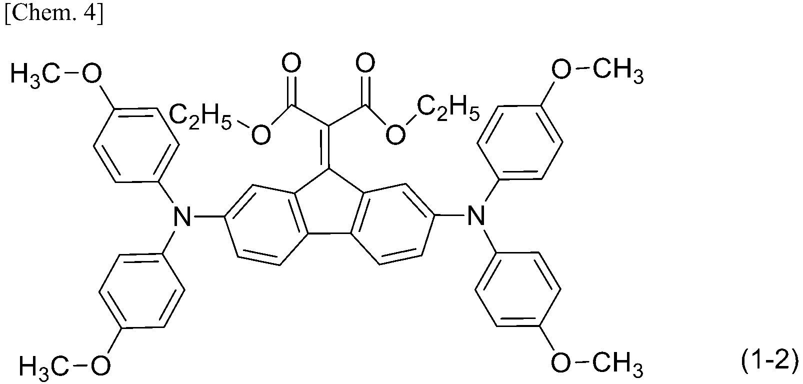

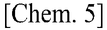

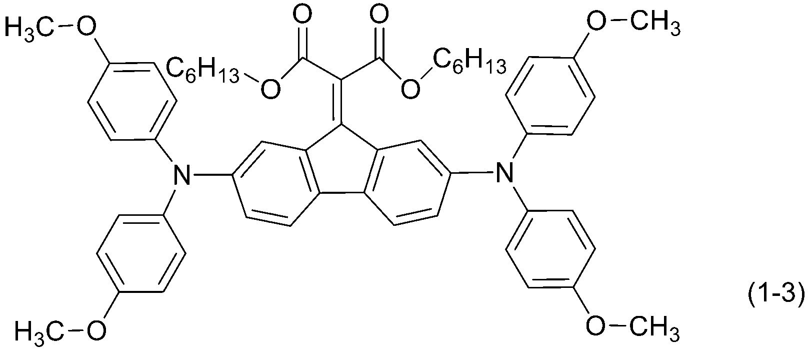

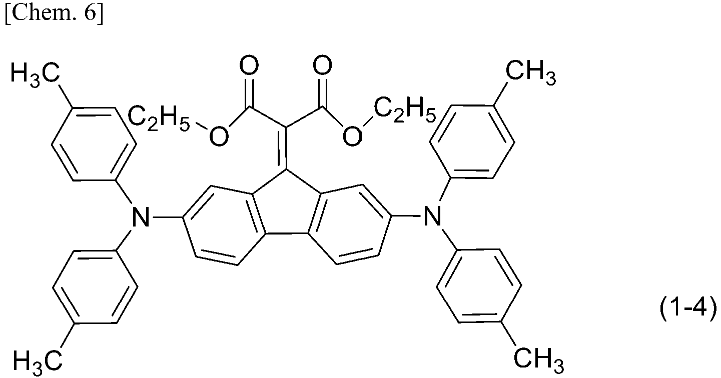

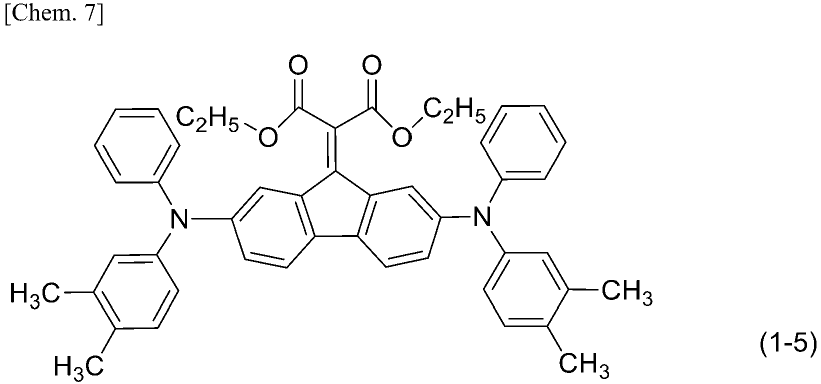

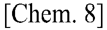

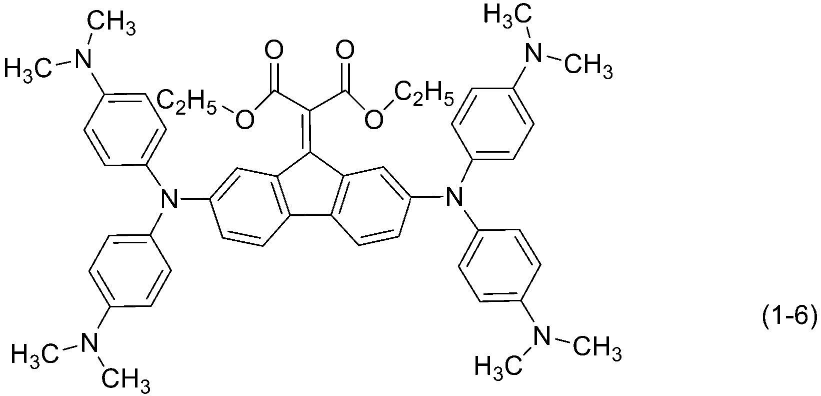

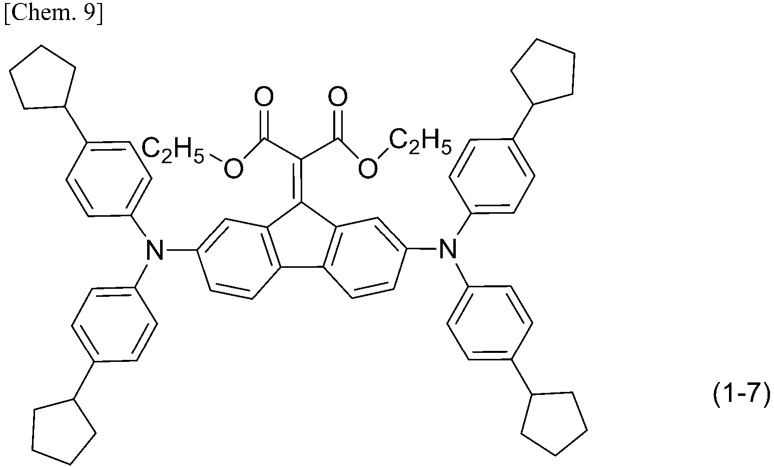

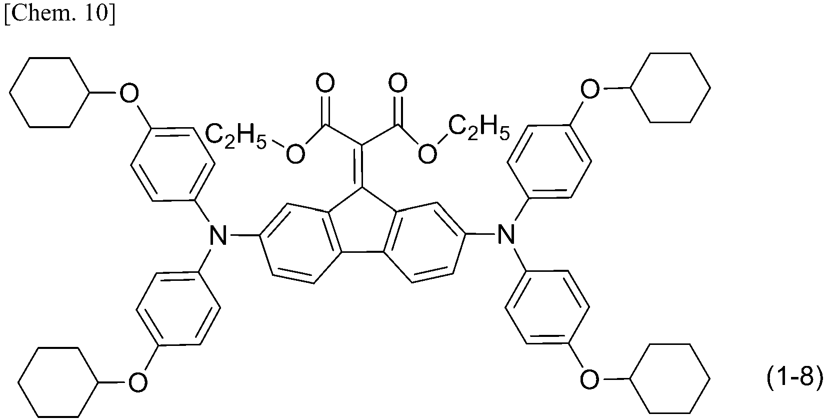

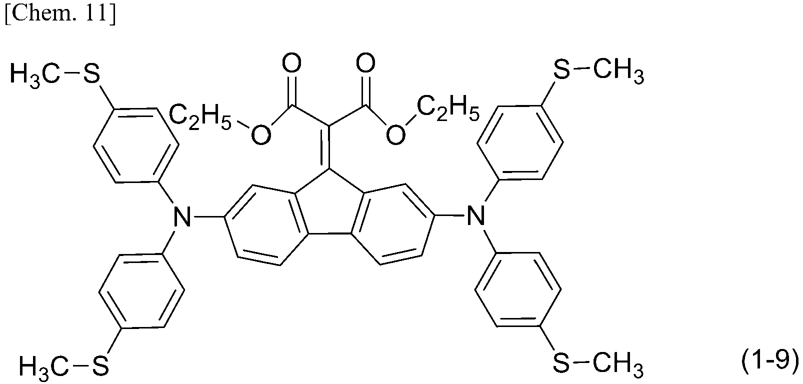

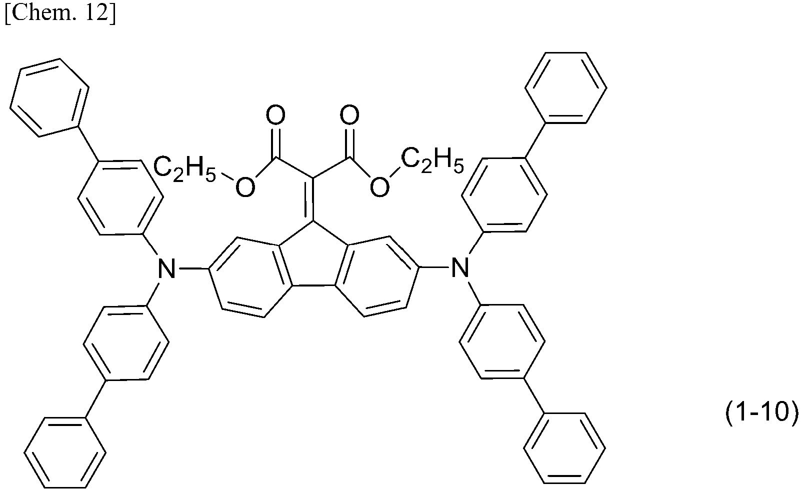

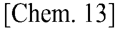

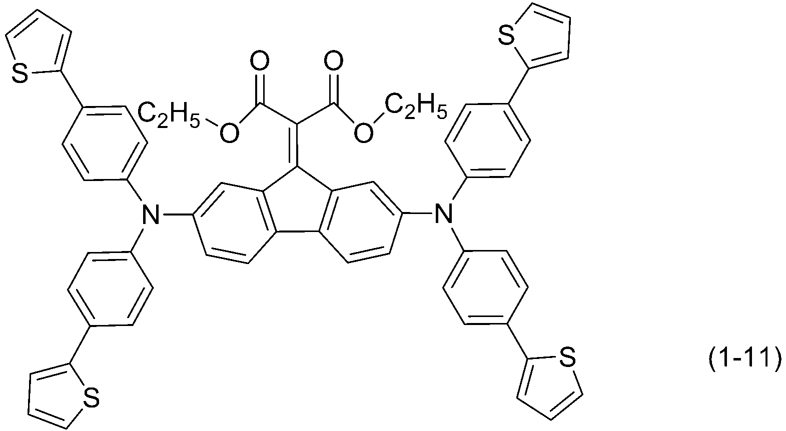

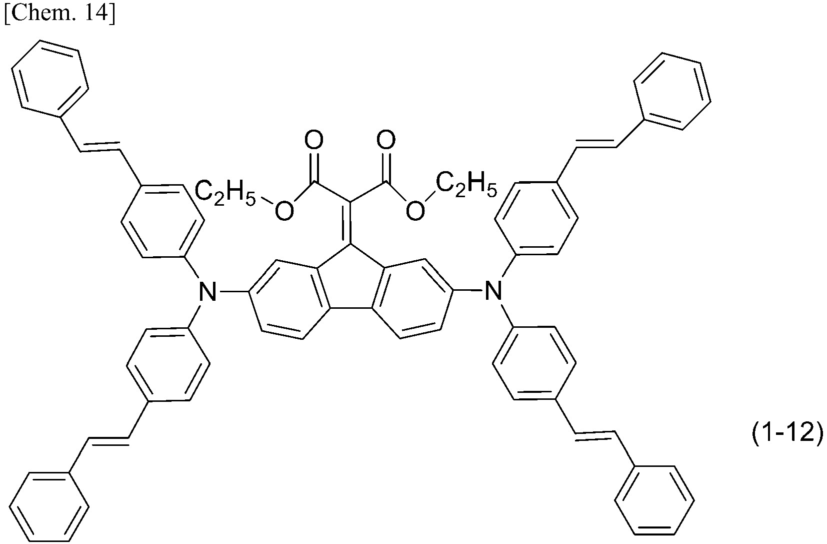

Hereinafter, the present invention will be described in detail by way of suitable embodiments. The present invention is not limited to the embodiments shown below, but covers those obtained by appropriately changing or modifying the embodiments shown below based on the general knowledge of persons skilled in the art without departing the gist of the present invention.

In this specification, "layer" means not only a layer having clear boundaries and flat and thin film-shaped layers, but also a layer with a gradient concentration of the element contained which gradually changes and a layer which may form a complex structure with another layer. The elemental analysis of the layer can be performed, for example, by performing TOF-SIMS/FE-TEM/EDS line analysis on a cross-section of a photoelectric conversion element and verifying the element distribution of specific elements.

Fig. 1 is a cross-sectional view schematically illustrating the configuration of a photoelectric conversion element according to one embodiment of the present invention. A second electrode 3, an electron transporting layer 4, a photoelectric conversion layer 5, a hole transporting layer 6, and a first electrode 7 are included on a substrate 2. One of the first electrode 7 and the second electrode 3 is a positive electrode, and the other thereof is a negative electrode. A current can be taken out by connecting the first electrode 7 and the second electrode 3 to an external circuit.

The photoelectric conversion layer 5 is excited by light entering through the substrate 2, the second electrode 3, and the electron transporting layer 4 or through the first electrode 7 and the hole transporting layer 6, and generates electrons or holes. In other words, the photoelectric conversion layer 5 generates a current between the first electrode 7 and the second electrode 3. The electron transporting layer 4 is a layer disposed between the photoelectric conversion layer 5 and either of the two electrodes 3 and 7, and need not to be formed in some cases. In one aspect, a plurality of electron transporting layers 4 and a plurality of photoelectric conversion layers 5 may form a laminate. Such a form can also be called a tandem structure. As shown in Fig. 2, the photoelectric conversion element may be prepared by forming the first electrode 7, the hole transporting layer 6, the photoelectric conversion layer 5, the electron transporting layer 4, and the second electrode 3 on the substrate 2 in this order.

Hereinafter, the photoelectric conversion element according to the present invention and the members included in the photoelectric conversion element will be described.

### [Photoelectric conversion element]

The photoelectric conversion element according to the present invention is a photoelectric conversion element including a first electrode, a second electrode, and a photoelectric conversion layer containing crystals with a perovskite structure, the photoelectric conversion layer being disposed between the first electrode and the second electrode, wherein a hole transporting layer is disposed between the photoelectric conversion layer and the first electrode. To improve the photoelectric conversion efficiency, the photoelectric conversion element may be laminated into a tandem type. The photoelectric conversion element laminated is not limited by the type of the photoelectric conversion element, such as perovskite solar batteries containing perovskite crystals in the photoelectric conversion layer, silicon solar batteries, and CIGS solar batteries.

Examples of a method of forming the layers in the photoelectric conversion element according to the present invention include coating methods and deposition methods. Examples of the coating methods include immersion coating, spin coating, spray coating, inkjet coating, meniscus coating, screen coating, roll coating, die coating, blade coating, curtain coating, and wire bar coating. The coating method is a method of preparing coating solutions for the layers described later, applying the coating solutions in desired order of layers, followed by drying. A desired method can be selected from these film forming methods according to the layers.

Hereinafter, the layers will be described.

### [Substrate]

The photoelectric conversion element according to the present invention 1 may include the substrate 2, and examples of the substrate include transparent glass substrates made of soda-lime glass or alkali-free glass, ceramic substrates, and transparent plastic substrates. When light is taken in from the side of the first electrode 7 in Fig. 1, an opaque material can be used in the substrate 2. When light is taken in from the side of the second electrode 3, the substrate 2 is formed from a transparent material.

### [Electrode]

The materials for the first electrode 7 and the second electrode 3 are not particularly limited, and conventionally known materials can be used. Examples thereof include metals such as gold, silver, titanium, and copper, sodium, sodium-potassium alloys, lithium, magnesium, carbon, aluminum, magnesium-silver mixtures, magnesium-indium mixtures, aluminum-lithium alloys, Al/Al₂O₃ mixtures, and Al/LiF mixtures. Examples of materials for transparent electrodes include conductive transparent materials such as CuI, ITO (indium tin oxide), SnO₂, AZO (aluminum zinc oxide), IZO (indium zinc oxide), GZO (gallium zinc oxide), FTO (fluorine-doped tin oxide), and ATO (antimony-doped tin oxide), and conductive transparent polymers. These materials may be used alone or in combination of two or more of these. For the first electrode 7 and the second electrode 3, at least one of the electrodes on the light entering side is a transparent electrode, while the other thereof may be a transparent electrode, may be an electrode also serving as a reflective layer formed from a light-reflective material, or may be a transparent electrode including a reflective layer on the side opposite to the light entering side. When the first electrode 7 is disposed on the light entering side, the second electrode 3 may be a transparent electrode and the substrate 2 may be a reflective layer. The transparent electrode may be a patterned electrode.

### [Photoelectric conversion layer]

The photoelectric conversion layer 5 contains crystals with a perovskite structure. The crystals in the perovskite structure used in the present invention are preferably represented by the following formula [2].

ABX₃ [2]

In the above formula [2], A represents an organic molecule or a monovalent cation of a metal atom, B represents a divalent metal cation, and X represents a monovalent halide anion.

A in the above formula [2] is preferably an organic molecule represented by CₚNₘHₙ (where p, m, and n are all a positive integer), for example. Specifically, examples of A include methylammonium and formamidium.

Inorganic atoms are preferably lithium, cesium, sodium, potassium, and rubidium, although not particularly limited. These organic molecules or inorganic atoms may be used alone or in combination of two or more of these.

When the cation of A included is too large to fit into the crystal of the three-dimensional perovskite structure, such a cation forms a crystal with a two-dimensional perovskite structure, a crystal with a 2.5-dimensional perovskite structure having properties derived from both of two-dimensional and three-dimensional perovskite structures, a two-layered crystal with three-dimensional and two-dimensional perovskite structures, or a crystal with a mixed structure of three-dimensional and two-dimensional perovskite structures, all of which function as a photoelectric conversion layer. The two-layered crystal with three-dimensional and two-dimensional perovskite structures indicates a crystal in which a crystal with a three-dimensional perovskite structure and that with a two-dimensional perovskite structure are laminated as independent and separate layers. The crystal with a mixed structure of three-dimensional and two-dimensional perovskite structures indicates a crystal with a structure in which regions or domains including both of a crystal with a two-dimensional or 2.5-dimensional layer-shaped perovskite structure and a crystal with a three-dimensional perovskite structure are mixed.

Preferably, the crystal with a two-dimensional perovskite structure or the crystal with a 2.5-dimensional perovskite structure are represented by the following formulae [3] to [5].

R'₂Aₙ₋₁BₙX_{3 n+1} [3]

R"Aₙ₋₁BₙX_{3 n+1} [4]

R‴AₙBₙX_{3 n+1} [5]

The formulae [3] to [5] form a RP (Ruddlesden-Popper) type perovskite structure, a DJ (Dion-Jacobson) type perovskite structure, and an ACI (Alternating cations in the interlayer) type perovskite structure, respectively.

In the formulae [3] to [5], R', R", and R‴ each represent a cation too large to fit to the three-dimensional perovskite structure, and for example, in an organic molecule, R', R", and R‴ are preferably represented by CₚNₘHₙ (where p, m, and n are all a positive integer). A may be unsubstituted or substituted, and specifically, A is preferably ethylammonium, propylammonium, n-butylammonium, n-hexylammonium, n-octylammonium, 1,6-hexanediammonium, iso-butylammonium, 3-(nonafluoro-tert-butyloxy)propylammonium, 1,3-propanediammonium, 1,5-pentamethylenediamine, 1,8-octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidium, propylammonium, propargylammonium, alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethaneammonium, 3-phenyl-2-propene-1-ammonium, phenylbutylammonium, 4-tert-butyl-benzylammonium, 3-(aminomethyl)piperidinium, or 4-(aminomethyl)piperidinium.

In the formulae [2] to [5], B represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Among these, lead, tin, and bismuth are preferred from the viewpoint of overlap of electron orbitals. These metal atoms may be used alone or in combination of two or more of these.

In the formulae [2] to [5], X represents a halogen atom, and examples thereof include chlorine, bromine, and iodine. These halogen atoms may be used alone or in combination of two or more of these. Among these, halogen atoms are preferred because a halogen contained in the structure is likely to cause the crystals with the above-mentioned perovskite structure soluble in an organic solvent, and enables application to an inexpensive printing method and the like. Furthermore, iodine is more preferred because the crystals with the perovskite structure have a narrower energy bandgap.

Specifically, the three-dimensional perovskite, the two-dimensional perovskite, and the mixed three-dimensional·two-dimensional perovskite are preferably MAPbI₃, FAPbCl₃, FAPbI₃, MAPbIₓBr₃₋ₓ, MAPbIₓCl₃₋ₓ, Cs0.05(MA0.17FA0.83)0.95Pb(I0.83Br0.17)3, Cs0.05FA0.88MA0.07PbI2.56Br0.44, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (4 FPEA)₂(FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I₁₆, (BA_{0.9}PEA_{0.1})₂MA₄Pb₅I₁₆, (BA_{0.9}PEA_{0.1})₂MA₃Pb₄I₁₃, (4 FPEA)₂MA₃Pb₄I₁₃, (4FPEA)₂MA₄Pb₅I₁₆(BA)₂MA₂Pb₃I₁₀, (BA)₂MA₃Pb₄I₁₃, (TEA)₂MA₂Pb₃I₁₀, (BA)₂MA₄Pb₅I₁₆, and (BA)₂MA₃Pb₄I₁₃. In the specific examples above, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethaneammonium, "TTDMA" represents thieno[3,2-b]thiophene-2.5-diyldimethaneammonium, "4 FPEA" represents 4-fluorinephenethylammonium, "BA" represents butylammonium, and "TEA" represents 2-thiophenethylammonium.

The crystals with the above-mentioned perovskite structure preferably have a structure of a cubic crystal system in which the metal atom B is body-centered, the organic molecule A is disposed at vertices, and a halogen atom or X is face-centered. Although details are not clear, it is inferred that when the crystals have such a structure, the direction of the octahedron in the crystal lattice can easily change, which increases the mobility of electrons in the crystals in the perovskite structure and improves the photoelectric conversion efficiency of the photoelectric conversion element.

The organic-inorganic perovskite compound used in the present invention is preferably a crystalline semiconductor. The crystalline semiconductor indicates a semiconductor whose scattering peak can be detected by measurement of X-ray scattering intensity distribution. When the organic-inorganic perovskite compound is a crystalline semiconductor, the mobility of electrons in the organic-inorganic perovskite compound increases, and the photoelectric conversion efficiency of the photoelectric conversion element is improved.

The photoelectric conversion layer according to the present invention has a thickness of preferably 5 nm or more to 2000 nm or less. If the photoelectric conversion layer has a thickness of 5 nm or more, the photoelectric conversion layer can sufficiently absorb light. If the photoelectric conversion layer has a thickness of 2000 nm or less, the generated charge can be transported to the electrodes. A more preferred lower limit is 50 nm and a more preferred upper limit is 1200 nm. A still more preferred lower limit is 100 nm and a still more preferred upper limit is 1000 nm.

### [Hole transporting layer]

In the present invention, the hole transporting layer 6 is disposed between the photoelectric conversion layer 5 and the first electrode 7 as illustrated in Figs. 1 and 2. As described above, the hole transporting layer 6 contains the compound represented by the formula (1).

For the purpose of improving the photoelectric conversion efficiency, a dopant (oxidizing agent) or a basic compound may be added.

The hole transporting layer according to the present invention preferably has a thickness of 10 nm or more to 1000 nm or less. If the hole transporting layer has a thickness of 10 nm or more, the hole transporting layer can sufficiently transport holes to the electrode. If the hole transporting layer has a thickness of 1000 nm or less, such a hole transporting layer is less likely to be resistance in hole transportation, thus increasing the photoelectric conversion efficiency. A more preferred lower limit of the thickness of the hole transporting layer is 50 nm and a more preferred upper limit is 500 nm.

### [Electron transporting layer]

In the photoelectric conversion element according to the present invention, the electron transporting layer 4 may be disposed between the second electrode 3 and the photoelectric conversion layer 5 as illustrated in Figs. 1 and 2.

Examples of a material for the electron transporting layer 4 include, but are not particularly limited to, N-type conductive polymers, N-type low-molecular organic semiconductors, N-type metal oxides, N-type metal sulfides, halogenated alkali metals, alkali metals, and surfactants. Specifically, examples thereof include cyano group-containing polyphenylenevinylene, boron-containing polymers, bathocuproine, bathophenanthrene, hydroxyquinolinatoaluminum, oxadiazole compounds, benzimidazole compounds, naphthalene tetracarboxylic acid compounds, perylene derivatives, phosphine oxide compounds, phosphine sulfide compounds, fluoro group-containing phthalocyanine, titanium oxide, zinc oxide, indium oxide, tin oxide, gallium oxide, tin sulfide, indium sulfide, and zinc sulfide.

A preferred lower limit of the thickness of the electron transporting layer 4 is 1 nm, and a preferred upper limit is 2000 nm. If the electron transporting layer has a thickness of 1 nm or more, the electron transporting layer can sufficiently block holes. If the electron transporting layer has a thickness of 2000 nm or less, the electron transporting layer is less likely to be resistance in electron transportation, thus increasing the photoelectric conversion efficiency. A more preferred lower limit of the thickness of the electron transporting layer 4 is 3 nm and a more preferred upper limit is 1000 nm. A still more preferred lower limit is 5 nm and a still more preferred upper limit is 500 nm.

### <Application Examples>

Application Examples are a photoelectric conversion apparatus, a moving body, and a construction material.

### [Photoelectric conversion apparatus]

The photoelectric conversion apparatus according to the present invention includes the photoelectric conversion element according to the present invention. The photoelectric conversion apparatus can be formed using a plurality of photoelectric conversion elements according to the present invention. When a plurality of photoelectric conversion elements is connected, the photoelectric conversion apparatus can also be called a photoelectric conversion cell or a photoelectric conversion module. In order to increase the output voltage, photoelectric conversion elements having different absorption wavelengths may be laminated. The photoelectric conversion apparatus includes the photoelectric conversion element according to the present invention and an inverter. The inverter may be a converter which converts DC to AC. The photoelectric conversion apparatus may include an electricity storage connected to the photoelectric conversion element. The electricity storage is not limited as long as it can store electricity. Examples thereof include secondary batteries, all-solid-state batteries, and electric double-layer capacitors using lithium ion or the like.

### [Moving body]

Fig. 3 is a perspective view schematically illustrating one embodiment of a moving body including the photoelectric conversion element according to the present invention. A moving body 30 includes a photoelectric conversion element 31 according to the present invention and a body 32 including the photoelectric conversion element 31. The photoelectric conversion element 31 is disposed in a position such that the photoelectric conversion element 31 can receive light external to the body 32. If the moving body 30 is a vehicle, the photoelectric conversion element 31 may be disposed on the roof thereof. The electric energy obtained by the photoelectric conversion element 31 may be used as the power of the moving body 30, or may be used as the power for another electric apparatus. The electric energy generated from the power of the moving body 30 may be used as the power of the photoelectric conversion element 31. If the moving body 30 is a vehicle, friction energy generated by braking may be converted to electric energy, which may be used to control the photoelectric conversion element 31.

The moving body 30 may be a vehicle, a ship, an airplane, or a drone, for example. The body 32 of the moving body 30 can be formed from any material, and is preferably formed from a strong material.

### [Construction material]

Fig. 4 is a perspective view schematically illustrating one embodiment of a construction material including the photoelectric conversion element according to the present invention. A construction material 40 may be a roof for a building. The construction material 40 according to the present embodiment includes the photoelectric conversion element 41 according to the present invention, a protective member 42 which protects the photoelectric conversion element 41, a heat dissipating member 43, and exteriors 44a and 44b.

The construction material 40 according to the present invention may include the heat dissipating member 43 having thermal conductivity higher than that of the photoelectric conversion element 41. If the photoelectric conversion element 41 is used as a roof or the like, the temperature of the photoelectric conversion element 41 may be increased by sunlight in some cases, which may reduce the photoelectric conversion efficiency. Use of the heat dissipating member 43 can suppress a reduction in photoelectric conversion efficiency. Examples of the heat dissipating member 43 include metals, alloys, liquid metals, and liquid resins.

The construction material 40 according to the present invention may include exteriors 44a and 44b. The exterior 44a may have a color different from that of the exterior 44b, or may have the same color as that of the exterior 44b. The exteriors 44a and 44b may be formed from the same member, or may be formed from different members. As the exterior member, coating materials and transparent substrates may be used. Preferred are those having low light absorption and high heat shielding properties.

### [Examples]

Hereinafter, the present invention will be more specifically described by way of Examples and Comparative Examples. The present invention is not limited by Examples below without departing from the gist thereof. To be noted, "parts" is mass-based unless otherwise specified in Examples below.

### <Synthesis of Exemplified compound (1-2)>

Exemplified compound (1-2) was synthesized by the following method.

2,7-Dibromo-9-fluorenone (3.38 g, available from Tokyo Chemical Industry Co., Ltd.), 4,4'-dimethoxydiphenylamine (5.04 g, available from Tokyo Chemical Industry Co., Ltd.), palladium acetate (89.8 mg, available from Tokyo Chemical Industry Co., Ltd.), 1,1'-bis(diphenylphosphino)ferrocene (332.6 mg, available from Tokyo Chemical Industry Co., Ltd.), cesium carbonate (9.77 g, available from Tokyo Chemical Industry Co., Ltd.), and toluene (300 mL) were placed into a reactor, and were stirred by heating under reflux under a nitrogen atmosphere for 18 hours. The reaction solution was left to stand to room temperature, followed by depressurized filtration, and washing with toluene. The filtrate was concentrated, and was refined by silica gel column chromatography (developing solvent: toluene), giving an intermediate product represented by the following formula (A) as a violet powder (yield: 44%).

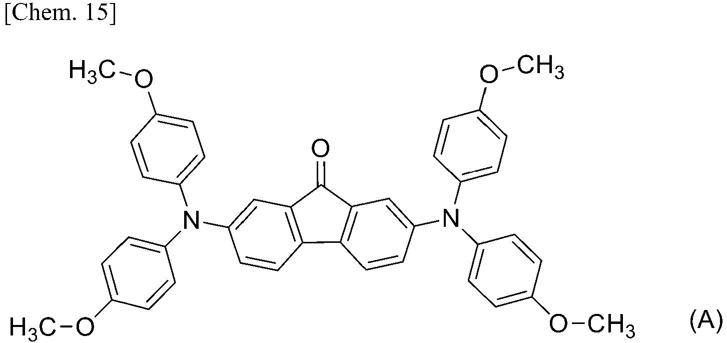

Subsequently, the intermediate product (1.27 g), diethyl malonate (1.60 g, available from Tokyo Chemical Industry Co., Ltd.), tetrahydrofuran (30 mL), and pyridine (5 mL) were placed into a reactor, and were cooled to 0°C with iced water. Then, titanium tetrachloride (3.79 g, available from Tokyo Chemical Industry Co., Ltd.) was added dropwise. After the addition, the reaction solution was left to stand to room temperature, and was stirred for 18 hours. After the end of the reaction, water and ethyl acetate were added to cause liquid separation. The organic layer was washed with water and saturated saline water, and was concentrated. The obtained crude product was refined by silica gel column chromatography (developing solvent: ethyl acetate/heptane = 1/3), giving Exemplified compound (1-2) as a violet powder (yield: 71%).

### <Production of photoelectric conversion element>

### (Example 1)

### [Formation of electron transporting layer]

A glass substrate with an ITO film was washed, and a tin(II) oxide coating solution having a concentration adjusted to 3% by mass was applied thereonto by spin coating. Then, the workpiece was heated at 150°C for 30 minutes to form an electron transporting layer in the form of a thin film having a thickness of 15 nm.

### [Formation of photoelectric conversion layer]

22.4 mg of lead bromide, 172 mg of formamidium iodide, and 576 mg of lead iodide were dissolved in 600 µL of N,N-dimethylformamide and 160 µL of dimethyl sulfoxide, followed by stirring for 1 hour (solution 1). Further, 389.72 mg of cesium iodide was dissolved in 1000 µL of dimethyl sulfoxide, followed by stirring for 1 hour (solution 2). Subsequently, the solution 2 (40 µL) was added to the solution 1 to prepare a coating solution for a photoelectric conversion layer. The coating solution was applied onto the electron transporting layer by spin coating to form a photoelectric conversion layer formed from Cs_{0.05}(FA_{0.83}MA_{0.17})_{0.95}Pb(I_{0.95}Br_{0.05})₃ and having a thickness of 500 nm.

### [Formation of hole transporting layer]

0.15 g of Exemplified compound (1-2) was dissolved in 2.2 g of chlorobenzene to prepare a coating solution 1 for a hole transporting layer. The coating solution was applied onto the photoelectric conversion layer by spin coating to form a hole transporting layer having a thickness of 200 nm.

### [Formation of first electrode]

A gold electrode having a thickness of 80 nm and an area of 0.09 cm² was formed on the hole transporting layer by a vacuum deposition method, obtaining a photoelectric conversion element.

### (Example 2)

A photoelectric conversion element was obtained in the same manner as in Example 1 except that in the preparation of the coating solution 1 for a hole transporting layer, Exemplified compound (1-2) was changed to a compound represented by Exemplified compound (1-3).

### (Example 3)

A photoelectric conversion element was obtained in the same manner as in Example 1 except that in the preparation of the coating solution 1 for a hole transporting layer, Exemplified compound (1-2) was changed to a compound represented by Exemplified compound (1-5).

### (Comparative Example 1)

A photoelectric conversion element was obtained in the same manner as in Example 1 except that in the preparation of the coating solution 1 for a hole transporting layer, Exemplified compound (1-2) was changed to a compound represented by the formula (A).

### (Comparative Example 2)

A photoelectric conversion element was obtained in the same manner as in Example 1 except that in the preparation of the coating solution 1 for a hole transporting layer, Exemplified compound (1-2) was changed to a compound represented by the following formula (B).

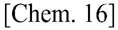

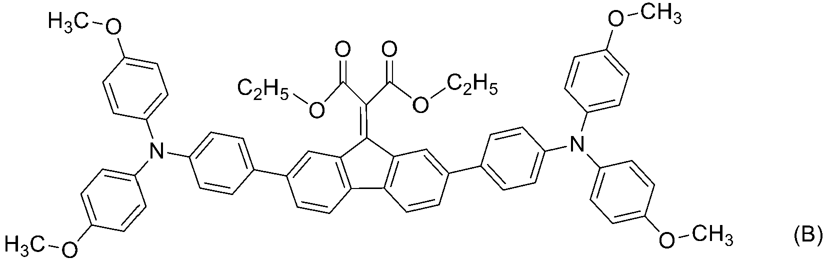

### (Example 4)

### [Formation of electron transporting layer]

A glass substrate with an ITO film was washed, and a tin(II) oxide coating solution having a concentration adjusted to 3% by mass was applied thereonto by spin coating. Then, the workpiece was heated at 150°C for 30 minutes to form an electron transporting layer in the form of a thin film having a thickness of 15 nm.

### [Formation of photoelectric conversion layer]

22.4 mg of lead bromide, 172 mg of formamidium iodide, and 576 mg of lead iodide were dissolved in 600 µL of N,N-dimethylformamide and 160 µL of dimethyl sulfoxide, followed by stirring for 1 hour (solution 1). Further, 389.72 mg of cesium iodide was dissolved in 1000 µL of dimethyl sulfoxide, followed by stirring for 1 hour (solution 2). Subsequently, the solution 2 (40 µL) was added to the solution 1 to prepare a coating solution for a photoelectric conversion layer. The coating solution was applied onto the electron transporting layer by spin coating to form a photoelectric conversion layer formed from Cs_{0.05}(FA_{0.83}MA_{0.17})_{0.95}Pb(I_{0.95}Br_{0.05})₃ and having a thickness of 500 nm.

### [Formation of hole transporting layer]

0.15 g of Exemplified compound (1-2) was dissolved in 2.2 g of chlorobenzene. 36 µL of an acetonitrile solution obtained by dissolving 0.2 g of lithium-bis(trifluoromethanesulfonyl)imide in 0.3 g of acetonitrile and 36 µL of t-butylpyridine were added to and mixed with the resulting chlorobenzene solution. Further, 58 µL of an acetonitrile solution obtained by dissolving 0.11 g of [tris(2-(1H-pyrazol-1-yl)-4-tert-butylpyridine)cobalt(III) tris(bis(trifluoromethylsulfonyl)imide)] in 0.3 g of acetonitrile was mixed to prepare a coating solution 2 for a hole transporting layer. The coating solution was applied onto the photoelectric conversion layer by spin coating to form a hole transporting layer having a thickness of 200 nm.

### [Formation of first electrode]

A gold electrode having a thickness of 80 nm and an area of 0.09 cm² was formed on the hole transporting layer by a vacuum deposition method, obtaining a photoelectric conversion element.

### <Evaluations>

### (Evaluation of photoelectric conversion efficiency)

A power supply (available from Keithley Instruments, Inc., 236 Model) was connected between the electrodes in the photoelectric conversion element obtained in Example 1. Using a solar simulator (available from Yamashita Denso Corporation) at an intensity of 100 mW/cm², the photoelectric conversion element was irradiated with certain light, and the generated current and voltage were measured to evaluate the photoelectric conversion efficiency.

Examples 2 to 4 and Comparative Examples 1 and 2 were evaluated likewise. The results are shown in Tables 1 and 2.

### [Table 1]

**Table 1**

| | Compound contained in hole transporting layer | Photoelectric conversion efficiency | Proportion to photoelectric conversion efficiency of Example 1 |
|---|---|---|---|
| Example 1 | Exemplified Compound (1-2) | 2.0% | 100% |
| Example 2 | Exemplified Compound (1-3) | 1.9% | 95% |
| Example 3 | Exemplified Compound (1-5) | 1.8% | 90% |
| Comparative Example 1 | (A) | 1.0% | 50% |
| Comparative Example 2 | (B) | 1.2% | 60% |

### [Table 2]

**Table 2**

| | Compound contained in hole transporting layer | Photoelectric conversion efficiency |
|---|---|---|
| Example 4 | Exemplified Compound (1-2) | 19.3% |

The present invention is not limited to the above embodiments, and can be subjected to a variety of alternations and modifications without departing from the gist and scope of the present invention. Accordingly, the following claims are attached to publicize the scope of the present invention.

This application claims priority based on Japanese Patent Application No. 2024-002687 filed on January 11, 2024, the entire contents of which are hereby incorporated by reference.

### [Reference Signs List]

1 photoelectric conversion element
2 substrate
3 second electrode
4 electron transporting layer
5 photoelectric conversion layer
6 hole transporting layer
7 first electrode

## Claims

1. A photoelectric conversion element comprising a first electrode, a second electrode, and a photoelectric conversion layer containing crystals with a perovskite structure, the photoelectric conversion layer being disposed between the first electrode and the second electrode,
wherein a hole transporting layer containing a compound represented by the following formula (1) is disposed between the photoelectric conversion layer and the first electrode: 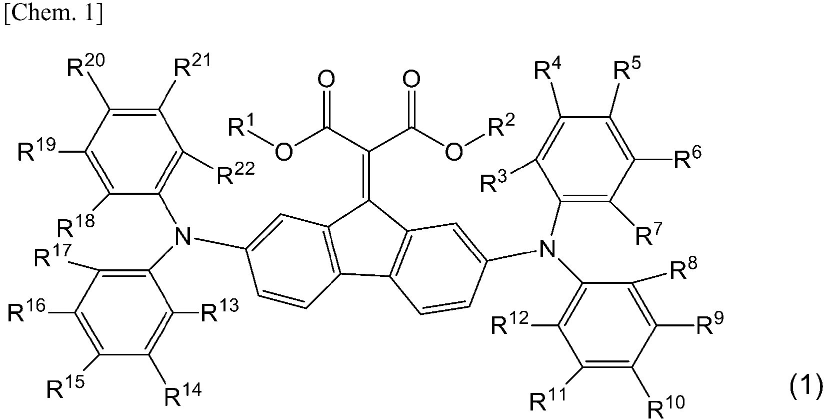
(wherein R¹ and R² each represent a linear or branched alkyl group having 1 to 6 carbon atoms, and R³ to R²² each independently represent a hydrogen atom, a trimethylsilyl group, a linear or branched alkyl group having 1 to 20 carbon atoms and optionally having a substituent, a linear or branched alkenyl group having 2 to 20 carbon atoms and optionally having a substituent, a cycloalkyl group having 3 to 10 carbon atoms and optionally having a substituent, an alkoxy group having 1 to 20 carbon atoms and optionally having a substituent, a cycloalkoxy group having 3 to 10 carbon atoms and optionally having a substituent, an alkylthio group having 1 to 18 carbon atoms and optionally having a substituent, an amino group having an alkyl group having 1 to 20 carbon atoms and optionally having a substituent, an aromatic hydrocarbon group having 6 to 36 carbon atoms and optionally having a substituent, or a heterocyclic group having 5 to 36 ring-forming atoms and optionally having a substituent; and the substituent which each of the functional groups optionally has is a halogeno group, a linear or branched alkyl group having 1 to 20 carbon atoms, an alkoxy group having 1 to 20 carbon atoms, an alkylthio group having 1 to 18 carbon atoms, an amino group having an alkyl group having 1 to 20 carbon atoms, an aromatic hydrocarbon group having 6 to 36 carbon atoms, or a heterocyclic group having 5 to 36 ring-forming atoms.)

2. The photoelectric conversion element according to claim 1, wherein in the formula (1), R³ to R²² each independently represent a hydrogen atom, a linear or branched alkyl group having 1 to 20 carbon atoms and optionally having a substituent, an alkoxy group having 1 to 20 carbon atoms and optionally having a substituent, or an amino group having an alkyl group having 1 to 20 carbon atoms and optionally having a substituent.

3. The photoelectric conversion element according to claim 1 or 2, wherein in the formula (1), R⁵, R¹⁰, R¹⁵, and R²⁰ each represent an alkoxy group having 1 to 20 or less carbon atoms and optionally having a substituent.

4. A photoelectric conversion apparatus comprising the photoelectric conversion element according to any one of claims 1 to 3.
